# EUROPEAN PATENT SPECIFICATION

(11) **EP 2 499 246 B1**
(45) Date of publication and mention of the grant of the patent: **04.03.2015**
(21) Application number: 09849292.9
(22) Date of filing: 11.09.2009
(51) Int. Cl.: C12N 15/10, C07H 21/00

(54) **METHOD FOR ISOLATING DNA**
VERFAHREN ZUR DNA-ISOLIERUNG
PROCÉDÉ POUR ISOLER DE L'ADN

(43) Date of publication of application: 19.09.2012
(73) Proprietor: Universiti Putra Malaysia, Selangor (MY)
(72) Inventor: HASSAN, Mohd, Ali, Selangor (MY); TABATABAEI, Meisam, Selangor (MY); ZAKARIA, Mohd, Rafein, Selangor (MY); RAHIM, Raha, Abdul, Selangor (MY); WRIGHT, Andre-Denis, G., St Lucia, QLD 4067 (AU); SHIRAI, Yoshihito, Kitakyushu 808 0196 (JP); ABDULLAH, Norhani, Selangor (MY); SHAMSARA, Mehdi, 14155-6343 Tehran (IR); SAKAI, Kenji, Higashi-ku Fukuoka 812-8581 (JP)
(74) Representative: Bates, Philip Ian
(86) International application number: PCT/MY2009/000143
(87) International publication number: WO 2011/031127

(56) References cited:
- EP-A1- 1 767 621
- EP-A1- 1 950 312
- EP-A2- 1 889 921
- WO-A2-2004/018673
- US-A- 5 128 247
- US-A1- 2005 084 878
- US-B1- 6 281 349
- ELSAS VAN J D ET AL: "SOIL DNA EXTRACTION AND ASSESSMENT OF THE FATE OF MYCOBACTERIUM CHLOROPHENOLICUM STRAIN PCP-1 IN DIFFERENT SOILS BY 16S RIBOSOMAL RNA GENE SEQUENCE BASED MOST-PROBABLE-NUMBER PCR AND IMMUNOFLOURESCENCE", BIOLOGY AND FERTILITY OF SOILS, BERLIN, DE, vol. 24, no. 2, 1 January 1997 (1997-01-01), pages 188-195, XP000946447, ISSN: 0178-2762, DOI: 10.1007/S003740050230
- TSAI Y-L ET AL: "RAPID METHOD FOR DIRECT EXTRACTION OF DNA FROM SOIL AND SEDIMENTS", APPLIED AND ENVIRONMENTAL MICROBIOLOGY, AMERICAN SOCIETY FOR MICROBIOLOGY, US, vol. 57, no. 4, 1 April 1991 (1991-04-01), pages 1070-1074, XP008044079, ISSN: 0099-2240
- LEMARCHAND K ET AL: "Optimization of microbial DNA extraction and purification from raw wastewater samples for downstream pathogen detection by microarrays", JOURNAL OF MICROBIOLOGICAL METHODS, ELSEVIER, AMSTERDAM, NL, vol. 63, no. 2, 1 November 2005 (2005-11-01), pages 115-126, XP027746392, ISSN: 0167-7012 [retrieved on 2005-11-01]
- BOURRAIN MURIEL ET AL: "DNA extraction from activated sludges", CURRENT MICROBIOLOGY, SPRINGER, NEW YORK, NY, US, vol. 38, no. 6, 1 June 1999 (1999-06-01), pages 315-319, XP002205479, ISSN: 0343-8651, DOI: 10.1007/PL00006809
- C. YEATES ET AL: "PCR amplification of crude microbial DNA extracted from soil", LETTERS IN APPLIED MICROBIOLOGY, vol. 25, no. 4, 1 October 1997 (1997-10-01), pages 303-307, XP055061657, ISSN: 0266-8254, DOI: 10.1046/j.1472-765X.1997.00232.x
- MILLER DN ET AL: "Evaluation and optimization of DNA extraction and purification procedures for soil and sediment samples", APPLIED AND ENVIRONMENTAL MICROBIOLOGY, AMERICAN SOCIETY FOR MICROBIOLOGY, US, vol. 65, no. 11, 1 January 1999 (1999-01-01), pages 4715-4724, XP002148457, ISSN: 0099-2240
- ORSINI AND V ROMANO-SPICA M: "A microwave-based method for nucleic acid isolation from environmental samples", LETTERS IN APPLIED MICROBIOLOGY, WILEY-BLACKWELL PUBLISHING LTD, GB, vol. 33, no. 1, 1 July 2001 (2001-07-01), pages 17-20, XP008161592, ISSN: 0266-8254, DOI: 10.1046/J.1472-765X.2001.00938.X [retrieved on 2001-12-20]
- OGRAM A ET AL: "The extraction and purification of microbial DNA from sediments", JOURNAL OF MICROBIOLOGICAL METHODS, ELSEVIER, AMSTERDAM, NL, vol. 7, no. 2-3, 1 December 1987 (1987-12-01), pages 57-66, XP023698903, ISSN: 0167-7012, DOI: 10.1016/0167-7012(87)90025-X [retrieved on 1987-12-01]
- MEISAM TABATABAEI1 ET AL: "Comparative study of methods for extraction and purification of environmental DNA from high-strength wastewater sludge", AFRICAN JOURNAL OF BIOTECHNOLOGY, ACADEMIC PRESS, US, vol. 9, no. 31, 2 August 2010 (2010-08-02) , pages 4926-4937, XP009169264, ISSN: 1684-5315

## Description

The present invention relates to the isolation of nucleic acid from environmental samples, and more particularly a direct isolation of high molecular weight genomic DNA from environment samples.

### BACKGROUND TO THE INVENTION

Isolated nucleic acid, and in particular, isolated high molecular weight DNA, has a variety of uses in molecular biology, biotechnology, environmental microbiology and clinical research. For example, isolated DNA is useful in a number of molecular biology techniques, including polymerase chain reaction (PCR), DNA hybridization, restriction enzyme digestion, DNA sequencing, and array-based experiments. With regard to biotechnology, isolated DNA is useful in the development of genetically engineered recombinant proteins and in identifying potential new therapeutic targets. In the clinical setting, isolated DNA is useful in the identification of genetic disorders and in the diagnosis of bacterial and/or viral infections.

As for environmental microbiology, isolated DNA is useful in forensic application of molecular technique which requires efficient extraction and purification of nucleic acids. Numerous DNA extraction methods have been developed and evaluated for acquiring genetic material from microorganisms present in soils and sediments, aerosols, water and other aqueous samples, as indigenous species or as organisms intentionally introduced to the environment.

Humic and fulvic acids are naturally occurring, polyelectrolytic, heterogeneous, organic substances that are generally dark brown in color, of relatively high molecular weight and, typically, resistant to degradation. They are found in water, air-borne organic materials, soils and sediments, and inhibit enzymatic (polymerase) activities characteristic of nucleic acid amplification techniques such as the polymerase chain reaction (PCR). They contain multiple functional groups such as phenolic and carboxylic moieties as well as hydrophobic components such as aliphatic or aromatic moieties.

Soils and sediments containing high organic carbon content also contain high levels of humic and fulvic acids. Humic acid concentrations from soil extractions vary according to soil/substrate types and for extraction methods, and in general, are found at concentrations ranging from 100-5000 mg/L. Accordingly, nucleic acid preparations extracted from soil and sediment can contain high levels of humic and fulvic acids, which in turn inhibit the amplification of the extracted nucleic acids. For example, standard PCR reactions have been inhibited by as little as 10 ng of humic acid. Additionally, the lysis and extraction method affects the quantity and quality of DNA recovered. The type of extraction method used may also preferentially yield DNA from one species relative to another species, and may also influence the amount of inhibitory substances co-extracted.

It has been shown that conventional methods for studying microbial diversity, such as plating on selective media, are unreliable, because only a small fraction of the bacterial species present in the natural habitat will grow on synthetic media. A newer approach is to estimate bacterial diversity by characterizing the DNA or RNA from a sample without cultivation procedures. This approach has been successfully applied on leaves, clays, coastal lagoons, biofilm and sludges. The description of bacterial species and their diversity in activated sludges is most important for the characterization of populations favoring floc structuration and, thus, efficient water purification.

It is now well established that many microorganisms in certain environmental samples cannot be readily cultivated with known isolation and incubation methods. Direct DNA extraction is a fast and simple method that uses physical, chemical, and, or enzymatic lysis for direct extraction of nucleic acids from different environmental samples.

United States patent number 6,261,842 discloses a method where cells in a suspension of environmental samples, for example soil suspension, are lysed and their DNA is extracted. This method provides a greater DNA yield, but has shortcomings. The genomic DNA recovered from lysis of an environmental sample may be derived from other non-microbial sources. Furthermore, this method results in DNA of less than 20 kb in size, and often containing substantial contaminants such as humic acid substances that interfere with subsequent manipulation of the DNA.

In Ogram method, a bead beater is used to disrupt cells following incubation in sodium dodecyl sulfate in sodium phosphate buffer. After centrifugation to remove glass beads and sediment particles, polyethylene glycol is added to precipitate DNA. Polyethylene glycol is removed by phenol-chloroform extraction. Following extraction, CsCl-ethidium bromide density gradient ultracentrifugation is used to concentrate and purify the DNA.

In Tsai and Olson method, sediments are treated with lysozyme, and cells are lysed by rapid freezing and thawing at -70 to 65[deg.]C. Following phenol-chloroform extraction, DNA is precipitated with isopropanol, and impurities are removed by gel filtration, as described by Moran et al. (1993).

The method of Jacobsen and Rasmussen differs from the above two methods in that cells are removed from sediments prior to lysis. A cation-exchange resin is used to break the attraction of the cells for sediment particles. Resin and sediment are removed by centrifugation, and cells are treated with lysozyme and pronase. CsCl- ethidium bromide density gradient ultracentrifugation is used to further purify the extracted DNA.

WO2004/018673 discloses a process for extracting total soil DNA. D1 discloses re-suspending soil in extraction buffer that contains Tris/HCl, Na-EDTA, Na-phosphate, NaCl and CTAB.

The above-mentioned methods have disadvantages in that the methods are time consuming and involve a high cost, especially if there is involvement of a large number of samples.

In view of the above, it is advantageous to provide a simple, reliable and inexpensive method in which a genomic DNA of high molecular weight and high quality can be isolated directly from environmental samples.

### SUMMARY OF THE INVENTION

The present invention provides a method for the isolation of nucleic acid from microbial cells in an environmental sample. The method includes preparing a suspension of the environmental sample, wherein the suspension consists of the environmental sample, water and ethylene diamine tetra acetic acid (EDTA), lysing the suspended sample with a buffered solution, adding sodium dodecylsulfate solution to the lysed suspended sample, carrying out solvent extraction and separation to obtain an aqueous phase, reacting the aqueous phase with solvents to generate an insoluble precipitate containing nucleic acid, and isolating the nucleic acid therefrom, thereby releasing high molecular weight nucleic acid pellets from the cells.

The method of the present invention is particularly useful in isolating high molecular weight DNA, typically genomic DNA, from water or liquid samples, aerosol samples (e.g. particulate material captured from the air soil samples on a filter or other capture material), as well as various other organic or environmental samples types, including stool samples, sludge samples, sewage samples, plant materials, and the like

Preferably, the invention provides a method for the isolation of DNA where at least 80%, and more preferably at least 90%, of the DNA has a molecular weight greater than 20 kb.

### BRIEF DESCRIPTION OF THE DRAWINGS

Figure 1 is a pulsed field agarose gel showing genomic DNA of different environmental samples isolated by a method according to the present invention; and
Figure 2 is a pulsed field agarose gel showing PCR amplification of methanogen 16S rRNA gene from DNA isolated using a method according to the present invention.

### DETAILED DESCRIPTION OF THE INVENTION

The present invention provides a novel method for the isolation of nucleic acid from a nucleic acid containing starting material. In one preferred aspect, the invention provides a method for the isolation of high molecular weight DNA, typically genomic DNA, from microbial cells in an environmental starting material. The environmental starting material may be, for example, water or liquid samples, aerosol samples (e.g. particulate material captured from the air soil samples on a filter or other capture material), as well as various other organic or environmental samples types, including stool samples, sludge samples, sewage samples, plant materials, and the like

Preferably, the invention provides a method for the isolation of DNA where at least 80%, and more preferably at least 90%, of the DNA has a molecular weight greater than 20 kb.

Accordingly, the invention provides a method for preparing a nucleic acid-containing extract in which humic and fulvic acids, as well as other polymerase inhibitors and impurities are removed to an extent that permits efficient nucleic acid amplification from the extract. The present inventors have discovered that by combining a separation step that includes filtration and centrifugation, whereby microbial cells are separated from each other and from other particles in the sample, and a step of suspension of separated pellets in ethylene diamine tetra acetic acid, microbial cells from environmental samples can be effectively recovered and their DNA isolated with improved purity while retaining high molecular weight characteristics.

The method of the present invention is inexpensive and convenient, i.e. avoids the use of cation-exchange resin, bead beating, or freeze-thawing, and rapid, i.e. typically isolates nucleic acids in about 5 hours. The isolated nucleic acid is in undamaged condition and has a high degree of purity. The method allows for the direct use of the nucleic acid as reagent in molecular biological reactions, for example in polymerase chain reaction (PCR).

As used herein, the term "microorganism" includes prokaryotic and eukaryotic microbial species from the Domains Archea, Bacteria and Eucarya, the latter including yeast and filamentous fungi, protozoa, algae, or higher Protista.

It should be understood that although the present invention is preferably used for the isolation of high molecular weight DNA, for example gDNA, it may also be used in the context of isolating other nucleic acids, for example, RNA, smaller molecular weight DNA, and the like.

### High Molecular Weight Nucleic Acid Isolation Method

One embodiment of the present invention is a method for isolating high molecular weight DNA from a DNA containing starting material. The method can be performed on a single sample or on a multitude of samples in a multi-well plate at the same time.

The method includes pre-extraction in order to remove organic pollutants present in the samples because trace levels of these pollutants act as inhibitors in the enzymatic analysis of DNA. Environmental samples contain particles and dirt, while activated sludge could be oily and greasy as well. So, an activated sludge sample, for example, is diluted with sterile water and filtered. Water is then added to facilitate the flow-through. The effluent is then centrifuged and the ions in the pellet are removed by chelation before undergoing enzymatic lysis.

The extraction method begins with addition of lysed buffer into the sample, which was then mixed and incubated in a water bath. Another solution of sodium dodecylsulfate was added to remove lipids and the sample was incubated. Then, proteins in the sample were removed by solvent extraction and centrifugation. The upper phase was collected and the last step was repeated to remove traces of proteins. Then, nucleic acids in the aqueous phase were precipitated using solvents and incubation and bulk nucleic acids were obtained by centrifugation. The DNA pellet obtained was washed with a solvent and re-centrifuged to remove salts.

The DNA pellet was dried, re-suspended in 1 ml of TE buffer, and stored at 4°C. Innovatively, for further purification of DNA extracts, DNA-binding membrane filter columns may be used, whilst binding, washing and elution steps may be carried out.

The present invention will now be described in detailed by way of examples.

### EXPERIMENTAL EXAMPLE

### Preparation of Sample

25 ml of activated sludge sample obtained from a 5000 L bioreactor was diluted with sterile water (1:1) and filtered using filter paper. Water was added to facilitate the flow-through. The effluent was then centrifuged at 8,200 x g for 15 minutes and the pellet was re-suspended in 10 ml of 0.5 M ethylene diamine tetra acetic acid (EDTA-Na), pH 8.0 and left at room temperature for 10 minutes before the enzymatic lysis.

### Extraction of DNA

10 ml of lysis buffer (10mM Tris, 1 mM EDTA with 2 mg/ml lysozyme, pH 8.0) was added and the samples were mixed and incubated at 37°C for at least 30 minutes in a water bath. A 10% (w/v) sodium dodecylsulfate solution was added to a final concentration of 0.5% and the samples were incubated at 70°C for 15 minutes.

Samples were mixed very gently with an equal volume of phenol/chloroform (1:1) and centrifuged at 2,000 x g for 10 minutes. The upper phase was collected and the phenol/chloroform step was repeated to remove traces of proteins.

Then, sodium acetate (3 M, pH 5.2) at 10% of the total volume was added to the supernatant and nucleic acids in the aqueous phase (≈22 ml) were precipitated with an equal volume of cold isopropanol at -20°C for 15 minutes. Bulk nucleic acids were obtained by centrifugation at 18,500 x g for 10 min at 4°C. The pellet was washed with 70% ethanol and re-centrifuged at 18,500 x g for 6 minutes at 4°C. The DNA pellet was vacuum dried for 1 hour at room temperature (25 ± 2°C), re-suspended in 1 ml of TE buffer [10 mM Tris-HCl, 1 mM EDTA (pH 8.0)], and stored at 4°C.

For further purification of DNA extracts, DNA-binding membrane filter columns may be used as an option which may be followed by binding, washing and elution steps.

### Validation

### (i) Gel Electrophoresis

DNA extracted from the activated sludge sample was subjected to electrophoresis for 45 minutes at 100 volts to determine the size of the DNA. The electrophoresis was also carried out for DNAs extracted from other samples that include activated sludge sample obtained from a recycling tank, activated sludge sample obtained from an anaerobic pond, a rumen liquor sample, a cow manure sample, a 45-day biocompost sample, and two cultivated methanogens, *Methanosaeta concilii* and *Methanothrix thermophila,* as positive controls.

### (ii) PCR primers and amplification conditions

To evaluate the method of the present invention, the extracted DNAs from activated sludge samples that were obtained from a 5000 L bioreactor, a recycling tank and an anaerobic pond, as well as the extracted DNAs from rumen liquor sample, cow manure sample, 45-day biocompost sample and two cultivated methanogens samples, i.e. *Methanosaeta concilii* and *Methanothrix thermophila,* as positive controls, were used directly in PCR reactions to amplify the 16S rDNA gene from Methanogenic bacteria. All the samples used contained methanogenic bacteria as a part of their normal microflora.

Each PCR mixture (25 µl) contained 0.5 µl of template, 2.5 µl PCR buffer (Fermentas, Hanover, Maryland, USA), 0.5 µl of 10 mM dNTPs, 2.5 µl of 25 mM MgCl₂, 0.5 µl of each methanogen primer Met86F (GCT-CAG-TAA-CAC-GTG-G) and Met1340R (CGG-TGT-GTG-CAA-GGA-G), and 0.2 µl of 5 U AmpliTaq DNA polymerase (Fermentas, Hanover, Maryland, USA). PCR was performed in a Perkin Elmer Gene Amp system 9600 in accordance with the following parameters: 35 cycles of 94°C for 40 seconds, 54°C for 50 seconds and 72°C for 90 seconds.

The PCR products were run on 1% agarose gel at 70 V for 45 minutes, stained with ethidium bromide and visualized under UV transillumination.

### (iii) DNA cloning and sequencing

The PCR products of activated sludge obtained from the 5000 L bioreactor and the recycling tank were cloned into pTZ57R vector according to the instructions of the manufacturer (Fermentas, Hanover, Maryland, USA). PCR products were sequenced on both strands using an ABI 3730 XL DNA Sequencer. Sequence data were analyzed using BLAST program.

### Results

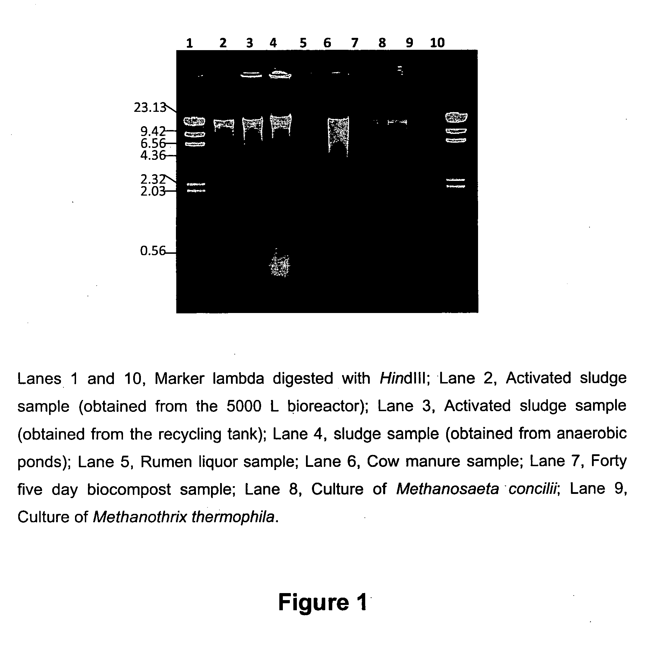

Figure 1 shows the electrophoresis pattern of the DNA extracted from activated sludge samples that were obtained from a 5000 L bioreactor, a recycling tank and an anaerobic pond, as well as the extracted DNAs from rumen liquor sample, cow manure sample, 45-day biocompost sample and two cultivated methanogens samples, i.e. *Methanosaeta concilii* and *Methanothrix thermophila,* as positive controls.

As shown in Figure 1, a high yield of high quality DNA with a ratio of A₂₆₀ to A₂₈₀ of more than 1.5 was obtained from various environmental samples.

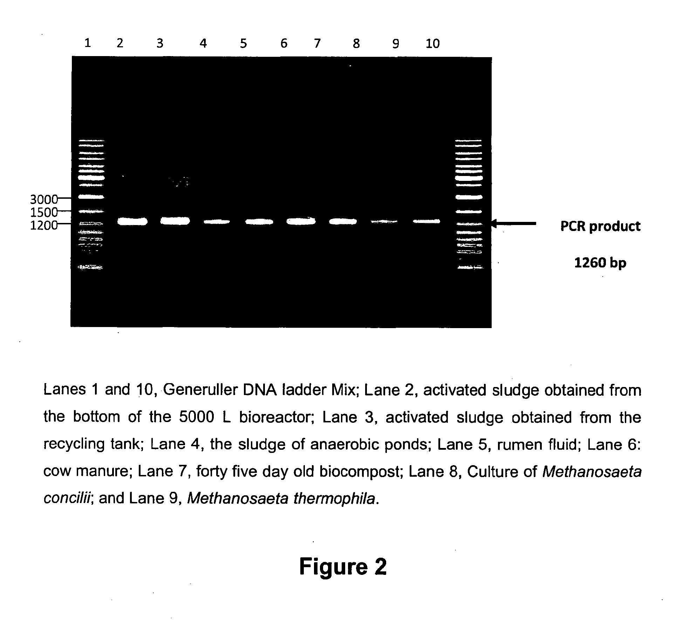

The DNA obtained from each sample was subjected directly in PCR reactions. Referring to Figure 2, a PCR product of 1260 bp is obtained in all of the six environmental samples, i.e. in lanes 2 to 7. The amplicon observed in the samples correspond to the amplicons of 1260 bp of 16S rDNA gene from the two cultivated methanogens samples, i.e. *Methanosaeta concilii* (lane 8) and *Methanothrix thermophila* (lane 9), *which act* as positive controls. This showed that all the samples used contained methanogenic bacteria as a part of their normal microflora. An expected amplicon of 1260 bp of 16S rDNA gene from the Methanogenic bacteria was successfully amplified from DNAs extracted from various environmental samples.

DNA sequence analysis of the cloned 16S rDNA genes confirmed that they were of methanogenic origin.

### COMPARATIVE EXAMPLE

For the purpose of comparative study, activated sludge samples were prepared and subjected to DNA extraction based on the three known methods described by Ogram *et al.* (1987), Tsai and Olson (1991) and Jacobsen *et al.* (1992).

Four different protocols that include a method of the present invention, the Ogram method, the Tsai method and the Jacobsen method were used for isolation of nucleic acids from activated sludge obtained from a recycling tank for the anaerobic treatment of palm oil mill effluent. For each procedure, four replicates were analyzed and four parameters (i.e. quantity, purity, fragmentation level of DNA and time) were compared to evaluate the performance of the different methods. The amount and purity of extracted DNA were assessed by absorbance at 260 nm and the ratio of absorbance at 260 and 280 nm. The DNA quality was considered reasonable when the ratio was >1.50. The occurrence of fragmentation of the extracted DNA was determined by electrophoresis of each DNA through a 1 % (w/v) agarose gel.

### Statistical analysis

A randomized complete block design with four replications was used for the analysis of DNA yield obtained by the four different methods. Mean comparison was carried out using Duncan's New Multiple Range Test (DNMRT). All statistical analyses were carried out using SAS version 9.1 (SAS Institute, Cary, NC).

### Results

**Table 1 below shows comparison of processing times, yields, and purities of DNA isolated from a method according to the present invention and three of the known methods, namely, Jacobsen method, Ogram method and Tsai method.**

| TABLE 1 | | | |
|---|---|---|---|
| Comparison of processing times, yields, and purities of DNA for different methods | | | |
| Extraction Method | Time¹ (Hours) | DNA yield (µg DNA/(g of sample)² | Purity ³ |
| Jacobsen | 22 | 0.33^{c} ± 0.05 | 1.34 |
| Ogram | 27 | 1.70^{b} ± 0.20 | 1.14 |
| Tsai | 11 | 0.65^{c} ± 0.03 | 1.12 |
| Present Invention | 5 | 2.75^{a} ± 0.03 | 1.65 |
| ¹Time required for extracting and purifying DNA from one activated sludge sample. | | | |
| ²Values are means of four independently extracted samples with standard error. | | | |
| ³ Ratio of A₂₆₀ to A₂₈₀. | | | |
| Means with different superscripts are significantly different (one-way ANOVA, Duncan's new multiple range test, P < 0.01) | | | |

The four extraction techniques differed in the time required to process a sample. The newly improved method took only 5 hours. The longest time was by Ogram method, which took 27 hours. The DNA yield also varied with extraction methods. The method according to the present invention had a significantly (P < 0.01) higher yield than the other methods (2.75 µg DNA/g of sample vs. 1.74, 0.62 and 0.35 by the Ogram, Tsai and Jacobsen methods respectively. So, the present DNA extraction method produced 161% and 423% more DNA than the commonly used methods of Ogram *et al.* (1987) and Tsai and Olson (1991).

The DNA purity, as indicated by the A260:A280 ratios differed among the methods. Only the method of the present invention gave a value of >1.50. The Tsai method showed the lowest DNA purity among the four protocols. In addition, the total cost incurred by the method of the present invention was about US$ 10 per sample, while the other three techniques were more expensive (i.e. about US$ 10, excluding the purification costs and Chelex resin).

### Conclusion

In all procedures previously established by Tsai, Ogram and Jacobsen, DNA samples were of low purity, apparently because of contamination with humic materials, but to different degrees; the Jacobsen samples had the lowest amount of contamination, and the Tsai samples had the greatest amount. The method of the present invention showed the best purity. It might be contributed to paper filtration, EDTA washing and the final cleaning step which not only reduced the humic materials contamination but also did not degrade the DNA. Amplification of the methanogen 16S rDNA gene from each sample demonstrated the high quality of the extracted DNA, as shown in Figure 2. No amplicons were observed when the samples did not undergo pre-extraction and the final cleaning steps. This may be due to the presence of PCR inhibitors, such as humic-acid-like substances and some metal ions found in crude DNA extracts as a small amount of humic-acid-like substances (e.g. 27 µg) or pure humic acid as low as 10 ng is sufficient to inhibit PCR.

As mentioned earlier when washing step by EDTA solution was omitted, no PCR amplification was observed. It has been reported that EDTA is a novel molecule to chelate or complex 2 and 3 valent cations such as Fe³⁺ which is a PCR-inhibitor in 1:1 metal-to-EDTA complexes. The importance of including the EDTA step is related to the fact of removing metal ions from the samples which could be potential PCR inhibitors. The final cleaning step to further purify the DNA extract took only 6 minutes and cost less than US$ 1 per sample, which allowed substantial cost savings in comparison with vastly used DNA binding membranes such as Elutip® Purification Minicolumns, which costs US$ 11.4 per column. Furthermore, the method of the present invention isolates nucleic acid is a rapid manner, i.e. five hours, compared to the other known methods. In conclusion, the present method produces nucleic acid of little shearing, clean enough to be amplified, less labor intensive than the other methods known in the art.

## Claims

1. A method for isolating nucleic acid from microbial cells in an environmental sample, including:
(i) preparing a suspension of the environmental sample, wherein the suspension consists of the environmental sample, water and ethylene diamine tetra acetic acid (EDTA);
(ii) lysing the suspended sample with a buffered solution;
(iii) adding sodium dodecylsulfate solution to the lysed suspended sample;
(iv) subjecting the product of step (iii) to solvent extraction and separation to obtain an aqueous phase;
(v) reacting the aqueous phase with solvents to generate an insoluble precipitate containing nucleic acid; and
(vi) isolating the nucleic acid therefrom, thereby releasing high molecular weight nucleic acid pellets from the cells.

2. A method according to claim 1, further comprising subjecting nucleic acid pellets of step (vi) to further nucleic acid purification.

3. A method according to claim 2, wherein further nucleic acid purification includes re-suspending the nucleic acid in a solvent and buffered solution, and isolating the nucleic acid therefrom.

4. A method according to claim 3, wherein the solvent is ethanol and the buffered solution is TE buffer.

5. A method according to claim 1, wherein the isolated nucleic acid is stored at 4°C.

6. A method according to claim 1, wherein the environmental sample is selected from the group consisting of water or liquid, soil, aerosol, stool, sludge, sewage samples and plant materials.

7. A method according to claim 1, wherein the buffered solution of step (ii) comprises ethylene diamine tetra acetic acid (EDTA) and lysozyme.

8. A method according to claim 1, wherein the solvent extraction of step (iv) is carried out using phenol and chloroform.

9. A method according to claim 1, wherein step (iv) is repeated more than once.

10. A method according to claim 1, wherein the solvents of step (v) include sodium acetate and isopropanol.

## Patentansprüche

1. Verfahren zum Isolieren von Nucleinsäure von mikrobiellen Zellen in einer Umweltprobe, das Folgendes beinhaltet:
(i) Herstellen einer Suspension der Umweltprobe, wobei die Suspension aus der Umweltprobe, Wasser und Ethylendiamintetraessigsäure (EDTA) besteht;
(ii) Lysieren der suspendierten Probe mit einer gepufferten Lösung;
(iii) Zugeben einer Natriumdodecylsulfatlösung zu der lysierten suspendierten Probe;
(iv) Aussetzen des Produkts aus Schritt (iii) einer Lösungsmittelextraktion und Trennung, um eine wässrige Phase zu erhalten;
(v) Zurreaktionbringen der wässrigen Phase mit Lösungsmitteln, um ein unlösliches Präzipitat zu erzeugen, das Nucleinsäure enthält; und
(vi) Isolieren der Nucleinsäure davon, so dass hochmolekulare Nucleinsäurepellets aus den Zellen freigesetzt werden.

2. Verfahren nach Anspruch 1, das ferner das Aussetzen der Nucleinsäurepellets aus Schritt (vi) einer weiteren Nucleinsäurereinigung beinhaltet.

3. Verfahren nach Anspruch 2, wobei die weitere Nucleinsäurereinigung das Resuspendieren der Nucleinsäure in einem Lösungsmittel und einer gepufferten Lösung und Isolieren der Nucleinsäure davon beinhaltet.

4. Verfahren nach Anspruch 3, wobei das Lösungsmittel Ethanol ist und die gepufferte Lösung TE-Puffer ist.

5. Verfahren nach Anspruch 1, wobei die isolierte Nucleinsäure bei 4°C gelagert wird.

6. Verfahren nach Anspruch 1, wobei die Umweltprobe aus der Gruppe bestehend aus Wasser- oder Flüssigkeits-, Boden- , Aerosol-, Stuhl-, Schlamm-, Abwasserproben und Pflanzenmaterialien ausgewählt wird.

7. Verfahren nach Anspruch 1, wobei die gepufferte Lösung aus Schritt (ii) Ethylendiamintetraessigsäure (EDTA) und Lysozym beinhaltet.

8. Verfahren nach Anspruch 1, wobei die Lösungsmittelextraktion aus Schritt (iv) mit Phenol und Chloroform durchgeführt wird.

9. Verfahren nach Anspruch 1, wobei Schritt (iv) mehr als einmal wiederholt wird.

10. Verfahren nach Anspruch 1, wobei die Lösungsmittel aus Schritt (v) Natriumacetat und Isopropanol beinhalten.

## Revendications

1. Procédé d'isolement d'un acide nucléique de cellules microbiennes dans un échantillon environnemental, comportant :
(i) la préparation d'une suspension de l'échantillon environnemental, la suspension consistant en l'échantillon environnemental, eau et acide éthylène diamine tétraacétique (EDTA) ;
(ii) la lyse de l'échantillon en suspension avec une solution tamponnée ;
(iii) l'ajout d'une solution de dodécyl sulfate de sodium à l'échantillon en suspension lysé ;
(iv) la soumission du produit de l'étape (iii) à une extraction et une séparation de solvant pour obtenir une phase aqueuse ;
(v) la mise en réaction de la phase aqueuse avec des solvants pour générer un précipité insoluble contenant l'acide nucléique ; et
(vi) l'isolement de l'acide nucléique du précipité, libérant ainsi des granules d'acide nucléique de poids moléculaire élevé depuis les cellules.

2. Procédé selon la revendication 1, comprenant en outre la soumission des granules d'acide nucléique de l'étape (vi) à une purification supplémentaire de l'acide nucléique.

3. Procédé selon la revendication 2, dans lequel la purification supplémentaire de l'acide nucléique comporte la mise en suspension à nouveau de l'acide nucléique dans un solvant et une solution tamponnée, et l'isolement de l'acide nucléique de cette suspension.

4. Procédé selon la revendication 3, dans lequel le solvant est l'éthanol et la solution tamponnée est un tampon TE.

5. Procédé selon la revendication 1, dans lequel l'acide nucléique séparé est stocké à 4°C.

6. Procédé selon la revendication 1, dans lequel l'échantillon environnemental est sélectionné dans le groupe consistant en échantillons d'eau, ou de liquide, terre, aérosol, selles, boue, eaux usées et matières végétales.

7. Procédé selon la revendication 1, dans lequel la solution tamponnée de l'étape (ii) comprend de l'acide éthylène diamine tétraacétique (EDTA) et du lysozyme.

8. Procédé selon la revendication 1, dans lequel l'extraction de solvant de l'étape (iv) est exécutée en utilisant du phénol et du chloroforme.

9. Procédé selon la revendication 1, dans lequel l'étape (iv) est répétée plus d'une fois.

10. Procédé selon la revendication 1, dans lequel les solvants de l'étape (v) comportent de l'acétate de sodium et de l'isopropanol.
